# FASCICULE DE BREVET EUROPEEN

(11) **EP 1 309 343 B1**
(45) Date de publication et mention de la délivrance du brevet: **02.10.2013**
(21) Numéro de dépôt: 01963076.3
(22) Date de dépôt: 08.08.2001
(51) Int. Cl.: A61K 39/385, A61K 39/00, A61P 35/00

(54) **VACCIN CONTRE L'IL-10 ISSUE DE TUMEURS MALIGNES**
IMPFSTOFF GEGEN IL-10 AUS MALIGNEN TUMOREN
VACCINES AGAINST IL-10 DERIVED FROM MALIGNANT TUMOURS

(30) Priorité: 09.08.2000 FR 0010480
(43) Date de publication de la demande: 14.05.2003
(73) Titulaire: NEOVACS, 75116 Paris (FR)
(72) Inventeur: ZAGURY, Jean-François, 75003 Paris (FR); BIZZINI, Bernard, 81000 Albi (FR); LE BUANEC, Hélène, 75005 Paris (FR); ZAGURY, Daniel, 75007 Paris (FR)
(74) Mandataire: Catherine, Alain
(86) Numéro de dépôt international: PCT/FR2001/002575
(87) Numéro de publication internationale: WO 2002/011759

(56) Documents cités:
- WO-A-00/03732
- WO-A-00/50071
- WO-A-92/22577
- WO-A-94/02167
- WO-A-96/10423
- WO-A-97/09064
- WO-A-99/15201
- ZAGURY D ET AL: "Toward a new generation of vaccines: The anti-cytokine therapeutic vaccines." PROCEEDINGS OF THE NATIONAL ACADEMY OF SCIENCES OF THE USA, vol. 98, no. 14, 3 juillet 2001 (2001-07-03), pages 8024-8029, XP002186083

## Description

La présente invention concerne l'utilisation de préparations vaccinales médicamenteuses à usage thérapeutique ou prophylactique destinées à traiter ou prévenir au sein des tumeurs malignes les dérèglements immunitaires en particulier l'immunosuppression et l'apoptose des cellules immunitaires ou vasculaires comme l'angiogénèse, induits par des facteurs extracellulaires, cytokines ou autres facteurs de régulation en particulier transcriptionnels, anormalement produits par les cellules cancéreuses ou les cellules stromales.

Les traitements conventionnels des cancers qu'ils soient d'origine virale, induits par des rétrovirus, ou l'EBV ou l'HPV ou encore les virus de l'hépatite, ou d'origine chronique, dus à l'amiante ou à des dérivés benzéniques, qu'ils soient de type épithélial (carcinomes) ou conjonctif (sarcomes) ou encore sanguin (lymphomes) comportent l'ablation chirurgicale des tumeurs le plus souvent associée à une chimiothérapie et/ou une radiothérapie.

Bien qu'efficaces pour certains cancers, particulièrement pris à des stades précoces, ces traitements souvent difficilement tolérés sont insuffisants et des récidives et des métastases compromettent l'évolution des malades.

C'est pourquoi lorsque les scientifiques dans les années 80 et 90 ont cloné et purifié des antigènes de tumeurs associés (TAA) ou spécifiques (TSA) aux cellules cancéreuses provenant de nombreuses tumeurs malignes (cancer du sein, de la prostate, colorectal, du col utérin ; lymphome ATL), de nombreuses expérimentations et essais cliniques de vaccination anti-cancer (Dvorak E. Experimental design for vaccine preparations against human malignant tumors. Med Hypotheses (1986) 20:429-52, Houghton AN. On course for a cancer vaccine. Lancet (1995) 345:1384-5, Herlyn D, Linnenbach A, Koprowski H, Herlyn M. Epitope-and antigen-specific cancer vaccines. Int Rev Immunol (1991) 7:245-57, Ostankovitch M, Choppin J, Guillet JG. Tumor cell antigenicity: cancers and vaccines. Rev Prat (1995) 45:1921-6, Zhu MZ, Marshall J, Cole D, Schlom J, Tsang KY. Specific cytolytic T-cell responses to human CEA from patients immunized with recombinant avipox-CEA vaccine. Clin Cancer Res (2000) 6:24-33, Tsunoda T, Tanimura H, Yamaue H, Tanaka H, Matsuda K. Tumor specific CTL therapy for advanced cancer and development for cancer vaccine. Hepatogastroenterology (1999) 46 :1287-92), utilisant comme antigènes les TAA et TSA ont été réalisés, visant à détruire spécifiquement les cellules malignes porteuses de ces antigènes grâce à l'action de cellules tueuses, particulièrement de lymphocytes cytolytiques (CTL), porteurs de récepteurs spécifiques, induits par la réaction immunitaire vaccinale.

Les essais cliniques utilisant de tels vaccins réalisés chez les malades porteurs de différentes tumeurs (mélanome, cancer du sein, cancer colorectal, cancer de la vessie, ...) ont permis d'établir les faits suivants :
- Les préparations vaccinales anti-cancer contenant les antigènes tumoraux (TAA ou TSA) présentés sous différentes formes ont été bien tolérées et n'ont généralement pas provoqué de complications régionales ou systémiques.
- De telles préparations vaccinales peuvent induire chez les malades une réponse immunitaire de type CTL (Tsunoda T, Tanimura H, Yamaue H, Tanaka H, Matsuda K. Tumor specific CTL therapy for advanced cancer and development for cancer vaccine. Hepatogastroenterology (1999) 1:1287-92, Schwaab T, Heaney JA, Schned AR, Harris RD, Cole BF, Noelle RJ, Phillips DM, Stempkowski L, Ernstoff MS. A randomized phase II trial comparing two different sequence combinations of autologous vaccine and human recombinant interferon gamma and human recombinant interferon alpha2B therapy in patients with metastatic renal cell carcinoma: clinical outcome and analysis of immunological parameters. J Urol (2000) 163:1322-7, Steller MA, Gurski KJ, Murakami M, Daniel RW, Shah KV, Celis E, Sette A, Trimble EL, Park RC, Marincola FM. Cell-mediated immunological responses in cervical and vaginal cancer patients immunized with a lipidated epitope of human papillomavirus type 16 E7. Clin Cancer Res (1998) 4:2103-9, susceptible in vitro de détruire spécifiquement les cibles cellulaires porteuses d'épitopes de TAA ou TSA complexés au Complexe Majeur d'Histocompatibilité.
- Par contre à ce jour aucun essai clinique de phase III n'a pu montrer que ces préparations vaccinales, visant à détruire spécifiquement les cellules cancéreuses par la différentiation de cellules tueuses, étaient efficaces.

Ainsi dès 1992, après que Levine (The p53 tumor suppressor gene and gene product. Princess Takamatsu Symp (1989) 20:221-30) ainsi que d'autres équipes scientifiques eurent montré que la protéine p53 native qui a des effets de réparation sur les brins d'ADN et des effets immunosuppressifs du cycle cellulaire ou un mutant de cette protéine était abondamment produite et accumulée dans les tumeurs malignes, le même Levine propose de réaliser une vaccination utilisant la protéine p53, apparaissant comme étant un antigène de tumeur associé (TAA). Celle-ci était présentée à la surface de cellules dendritiques (DC) ou adjuvantée dans un vecteur bactérien (type BCG) de manière à induire une réponse immunitaire de type CTL dirigée contre les cellules cancéreuses (Voir aussi WO-A-94/02167).

A l'appui de cette demande de brevet, des publications scientifiques montrent le rôle bénéfique des cellules tueuses et le rôle péjoratif des anticorps spécifiques dans l'évolution des tumeurs malignes (Theobald M, Biggs J, Dittmer D, Levine AJ, Sherman LA. Targeting p53 as a general tumor antigen. Proc Natl Acad Sci U S A (1995) 92:11993-7, Roth J. et al, p53 as a target for cancer vaccines: recombinant canarypox virus vectors expressing p53 protect mice against lethal tumor cell challenge, 1996, Proc Natl Acad Sci USA.; 93:4781-6).

A la suite de Levine, d'autres équipes modifiant le vecteur ou l'adjuvant de l'immunogène p53 ont déposé une dizaine de demandes de brevets sur l'utilisation de nouvelles présentations galéniques de vaccin anti p53 visant également à induire la formation de cellules tueuses CTL ciblant les cellules cancéreuses exprimant la protéine p53.

Les essais expérimentaux associés à ces vaccins anti p53 ont montré l'innocuité et l'immunogénicité évaluée par l'apparition de cellules tueuses anti p53. Plus, le seul essai clinique de vaccination anti p53 réalisé et publié a confirmé l'innocuité et l'immunogénicité du vaccin. Cependant aucun essai de phase III n'a pu valider l'efficacité de cette stratégie vaccinale.

La demanderesse a découvert avec étonnement que l'immunosuppression et l'angiogénèse du micro environnement des cellules infectées par certains virus tel le VIH-1 et du micro environnement des cellules cancéreuses apportent une explication rationnelle à l'absence d'efficacité de ces stratégies vaccinales car ces stratégies antérieures ciblent la cellule cancéreuse et non le dérèglement de son micro environnement.

Or, alors que jusqu'à présent les traitements ont tous visé à tuer directement les cellules cancéreuses elles-mêmes, c'est à dire les cellules parenchymateuses, la demanderesse a trouvé qu'il était autant ou même plus judicieux de lutter contre les molécules produites dans le micro environnement extra cellulaire (stromal) de la tumeur et favorisant le développement de cette dernière.

Rappelons que tout tissu ou tumeur est formée de cellules parenchymateuses qui baignent dans un micro environnement appelé stroma. Ce stroma est lui-même constitué de cellules stromales (qui peuvent être des cellules immunitaires, endothéliales, ou fibroblastiques) et d'un milieu extra cellulaire.

Les travaux de la demanderesse ont montré en effet que des facteurs solubles secrétés par les cellules infectées par le VIH-1, en particulier la protéine Tat ou par les cellules immunitaires de patients infectés par le VIH en particulier l'IFNα et le TGFβ ou produits par des cellules cancéreuses, telles la protéine E7 de l'HPV dans le cancer du col utérin ou la protéine Tax du HTLV1 dans les leucémies ATL ou la protéine p53 dans le cancer colorectal , avaient des propriétés immunosuppressives susceptibles d'inhiber les réactions immunitaires cellulaires au sein des tumeurs et de ce fait expliquaient l'inefficacité des vaccins antérieurs.

L'étude bibliographique a permis de conforter ces observations de la demanderesse, en confirmant la présence de facteurs immunosuppressifs relâchés dans le milieu extracellulaire de tumeurs malignes :
Certains de ces facteurs non encore identifiés ont été produits par
   - des cellules de cancer colorectal (Ebert EC, Roberts AI, O'Connell SM, Robertson FM, Nagase H. Characterization of an immunosuppressive factor derived from colon cancer cells. J Immunol. (1987) 138:2161-8 ou Remacle-Bonnet MM, Pommier FJ, Kaplanski S, Rance RJ, Depieds RC. Inhibition of normal allogenic lymphocyte mitogenesis by a soluble inhibitor extracted from human colonic carcinoma. J Immunol (1976) 117:1145-51,
   - des cellules de glioblastome (29-Fontana A, Hengartner H, de Tribolet N, Weber E. Glioblastoma cells release interleukin 1 and factors inhibiting interleukin 2-mediated effects. J Immunol. (1984) 132:1837-44),
   - des mélanomes (30.Hersey P, Bindon C, Czerniecki M, Spurling A, Wass J, McCarthy WH. Inhibition of interleukin 2 production by factors released from tumor cells. J Immunol. (1983) 131:2837-42), ou
   - des ascites malignes (Tamura K, Shibata Y, Matsuda Y, Ishida N. Isolation and characterization of an immunosuppressive acidic protein from ascitic fluids of cancer patients. Cancer Res. (1981) 41:3244-52, Oh SK, Moolten FL. Non specific immunosuppressive factors in malignant ascites : further characterization and possible relationship to erythrocyte receptors of human peripheral T cells. J Immunol. (1981) 127:2300-7).

D'autres facteurs de régulation transcriptionnelle, comme rapporté plus haut, sont d'origine cellulaire telle la protéine p53, accumulée dans certaines tumeurs malignes, en particulier colorectales (Remvikos Y, Tominaga O, Hammel P, Laurent-Puig P, Salmon RJ, Dutrillaux B, Thomas G. Increased p53 protein content of colorectal tumours correlates with poor survival. Br J Cancer 1992 66:758-64, Gan H, Ouyang Q, Wang Y. Expression of p53 protein in colorectal cancer and its relationship to cell proliferative activity and prognosis. Chung Hua Chung Liu Tsa Chih (1996) 18:244-6). La protéine p53, relâchée par transport actif par des voies de sécrétion n'utilisant pas le signal peptide ou par diffusion passive est présente dans le milieu extracellulaire, et elle a été isolée par chromatographie sur fibre de verre à partir de sérum de cancéreux (Zusman I, Sandler B, Gurevich P, Zusman R, Smirnoff P, Tendler Y, Bass D, Shani A, Idelevich E, Pfefferman R, Davidovich B, Huszar M, Glick J. Comparative study of the role of serum levels of p53 antigen and its tumor cell concentration in colon cancer detection. Hum Antibodies Hybridomas. (1996) :123-8, Sandler B, Smirnoff P, Tendelr Y, Zinder O, Zusman R, Zusman I. Specificity of polyclonal anti-p53 IgG for isolation of the soluble p53 antigen from human serum. Int J Mol Med. 1998 1:767-70).

Des cytokines, telles le TGFβ notoirement immunosuppressif; le VEGF facteur de croissance angiogénique, l'IL 6 pro-inflammatoire ou l'IL 10 également immunosuppressive, sont anormalement secrétées et relâchées dans le milieu extracellulaire de certaines cellules cancéreuses. La demanderesse a elle-même montré que les cellules de lignée cancéreuses SIHA, tout comme les cellules DU145 du cancer de la prostate et les cellules MT2 de lignées leucémiques produisent anormalement et relâchent dans le milieu extracellulaire des cytokines telles le VEGF et/ou l'IL 6 tandis que les cellules RAJI de lignées leucémiques secrètent dans le milieu extracellulaire de l'IL 10.

Dans ce contexte, la présente invention a pour objet l'utilisation comme médicament anticancéreux de nouvelles préparations vaccinales dénuées de toxicité et destinées à neutraliser l'IL-10 produite dans le compartiment stromal extra cellulaire en excès par les cellules cancéreuses ou stromales de tumeurs malignes.

Les exemples de vaccins anti-cytokines décrits dans EP-591.281 concernaient particulièrement des vaccins anti-IFNα utilisés contre le SIDA et d'autres affections immunitaires. Les exemples d'immunogènes décrits dans WO-A-00/03732 dérivaient de facteurs de régulation d'origine virale telles les protéines E7 du HPV 16, Tax du HTLV-1 et du Tat du HIV-1.

Dans ces nouvelles préparations vaccinales non toxiques, l'immunogène est présenté sous une forme galénique permettant d'induire une réaction immunitaire induisant préférentiellement des anticorps de classe IgG et/ou IgA capables d'antagoniser localement l'IL-10 anormalement présente dans le milieu extracellulaire des tumeurs et d'en inhiber les effets.

La présente invention propose l'utilisation comme médicament anticancéreux de vaccins dirigés particulièrement contre la protéine IL 10, cytokine immunosuppressive majeure.

Ces vaccins visent à induire une réaction immunitaire avec formation d'anticorps de classe IgG (pour tous les cancers) et surtout de classe IgA (pour les cancers épithéliaux) de manière à neutraliser localement, au sein de la tumeur, l'IL-10 et à en bloquer les effets, permettant ainsi à l'immunité naturelle ou à un vaccin dirigé contre les antigènes TAA ou TSA de fonctionner normalement et d'éliminer les cellules malades.

C'est pourquoi la présente demande a pour objet un vaccin caractérisé en ce qu'il renferme à titre de principe actif un immunogène qui est un l'IL-10 ou qui dérive de l'IL-10, ainsi qu'un excipient pharmaceutiquement acceptable permettant l'induction d'une réaction immunitaire systémique ou mucosale avec formation d'anticorps de classe IgG ou IgA sécrétoire dirigée contre le facteur natif.

Dans des conditions de mise en oeuvre, l'immunogène dérive de l'IL-10 par traitement chimique, physique, par mutation génétique, par conditionnement adjuvant ou est le produit d'une vaccination génétique (vaccin à ADN) ou est un fragment protéique ou peptidique d'un tel facteur ou encore dérive d'un tel fragment protéique ou peptidique.

L'immunogène sera de préférence couplé à une protéine porteuse.

En effet la demanderesse a découvert qu'une telle mesure augmente le nombre des sites auxiliaires (helper) et de ce fait augmente la réponse anticorps neutralisant le facteur extra cellulaire ciblé.

Dans encore d'autres conditions préférentielles de mise en oeuvre l'immunogène dérive de l'IL-10 par couplage à une protéine porteuse qui est le KLH.

Des traitements de l'immunogène qui ont été décrits dans WO-A-00/03732 peuvent être utilisés dans la présente invention pour l'obtention d'un vaccin dénué de toute toxicité et en particulier dépourvu de tout caractère immunosuppressif. Mais les immunogènes peuvent aussi être utilisés à l'état natif.

Les traitements chimiques consistent par exemple à détoxiquer la protéine native ou recombinante par un traitement aux aldéhydes notamment le formaldéhyde, aldéhyde monofonctionnel et donc n'agissant pas par couplage de molécules, conformément à la détoxication des toxines tétaniques ou diphtériques, ou consistent encore en des traitements bloquant les groupements sulfidryles, tels la carboxamidation, la maléimidation ou la carboxyméthylation ou en tout autre traitement bloquant d'autres résidus aminés comme décrit dans des demandes antérieures de la demanderesse.

Dans toujours d'autres conditions préférentielles de mise en oeuvre, l'immunogène est un mutant du facteur natif ou un fragment du facteur natif.

On pourra utiliser un mutant du facteur possédant au moins 70 %, de préférence au moins 80 % et tout particulièrement au moins 90 %, d'homologie avec le facteur protéique natif ou encore un fragment protéique ou peptidique du facteur. Dans le cas d'un peptide, celui-ci sera de préférence porté par une protéine porteuse tel le KLH ou le toxoïde tétanique. On pourra avantageusement utiliser aussi une protéine porteuse dans le cas du facteur protéique natif ou encore d'un fragment protéique de celui-ci.

Les produits décrits ci-dessus utilisés comme immunogènes, à l'exception des facteurs natifs, sont nouveaux, au moins pour la plupart d'entre eux. Ils entrent donc dans le cadre de l'invention.

Les traitements physiques peuvent être réalisés par la chaleur, les radiations U.V., les rayons X ou le contact avec une atmosphère riche en O₂. Ces traitements physiques générant des modifications intramoléculaires entre radicaux chimiques (groupement thiols par exemple), peuvent de manière appropriée changer la conformation de la molécule, l'inactiver fonctionnellement tout en conservant ses propriétés immunogènes.

Les modifications génétiques peuvent être obtenues par ingénierie génétique opérant des insertions, des délétions ou des substitutions de résidus. Les mutants génétiques pourront ou non subir un traitement chimique et/ou physique complémentaire. Les protéines modifiées ci-dessus peuvent par exemple être préparées à partir d'une protéine ayant une séquence identique ou similaire à une séquence peptidique d'un facteur ci-dessus. Tous ces procédés sont bien connus de l'état de la technique.

Un vaccin à ADN (vaccination génétique) pourra comprendre un plasmide comportant un gène promoteur d'expression comme celui du CMV et le gène codant pour un immunogène défini ci-dessus (facteur natif ou dérivé dont fragments).

Par "dérivent" ou "dériver" de facteurs immunosuppressifs / apoptotiques / angiogéniques, l'on entend aussi que le composé immunogène peut être constitué de la totalité ou d'un fragment de la protéine de départ ou encore peu notamment être couplé à une protéine porteuse comme le KLH (keyhole limpet hemocyanin) ou le tétanos toxoïde, directement ou de préférence par un réactif bifonctionnel de couplage.

Il peut comporter une ou plusieurs modifications dans les acides aminés de cette protéine ou fragment telles que des délétions, substitutions, additions, ou fonctionnalisations telles qu'acylation d'acides aminés, dans la mesure où ces modifications restent dans le cadre précisé ci-dessus (absence de toxicité, caractères immunologiques). Par exemple, en général le remplacement d'un résidu leucine par un résidu isoleucine ne modifie pas de telles propriétés; les modifications doivent généralement concerner moins de 40% d'acides aminés, notamment moins de 30% de préférence moins de 20% et tout particulièrement moins de 10% du facteur protéique. Il est important que la protéine ou fragment modifié ne soit pas dénaturé comme on peut le faire par exemple par un traitement physique comme la chaleur afin de préserver ses sites conformationnels pour que les anticorps induits par les dérivés modifiés soient actifs vis à vis de la protéine native.

De manière générale, en ce qui concerne les modifications, l'homologie ou la similitude entre l'immunogène modifié et la protéine ou partie de protéine immunosuppressive native, ainsi que les dimensions du composé immunogène, de même que les modalités d'utilisation, ou de couplage du composé immunogène selon l'invention à une protéine immunogène telle que le toxoïde tétanique, on peut en particulier se référer à WO-A-86/06 414 ou à EP-A-0.220.273 ou encore à PCT/US.86/00831, équivalents.

Un fragment peut comporter de 8 à 110 acides aminés par exemple, de préférence de 20 à 110, notamment de 12 à 60, particulièrement de 25 à 60, plus particulièrement de 12 à 40 et tout particulièrement de 30 à 50 acides aminés. Un tel fragment peut comporter aussi du ou des côtés C ou N terminal de 1 à 5 acides aminés supplémentaires c'est-à-dire différents du segment d'origine. Un fragment doit en outre comporter une cystéine au moins pour pouvoir faire l'objet de la carboxyméthylation.

Un fragment protéique pourra comporter la totalité des acides aminés de la séquence native, délétée de moins de 25 acides aminés, de préférence moins de 15 acides aminés, particulièrement moins de 10 acides aminés et tout particulièrement moins de 5 acides aminés, voire même un seul acide aminé.

Pour le conditionnement adjuvant, l'immunogène pourra notamment être inclus dans une émulsion eau dans huile, en utilisant par exemple l'ISA 51.

Une préparation vaccinale contenant l'immunogène anti immunosuppressif / apoptotique / angiogénique pourra être administrée sous une forme galénique appropriée à induire une réponse immunitaire de type systémique par voie Intra Musculaire (IM), Sous Cutanée (SC), Intra Dermique (ID) ou de type mucosal par voies intra nasale, orale, vaginale ou rectale.

Une préparation vaccinale contenant l'immunogène anti immunosuppressif / apoptotique *l* angiogénique pourra également contenir d'autres immunogènes, tels les antigènes TAA ou TSA de cancer ou des adjuvants telles des cytokines ou des protéines d'entérotoxines, type CTB ou Lt mutant (LTµ) (Freytag LC, Clements JD, Bacterial toxins as mucosal adjuvants Curr Top Microbiol Immunol; (1999) 236:215-36).

Une préparation galénique à visée systémique, administrée par voie SC, IM, ID, pourra être une émulsion eau renfermant l'immunogène, dans huile, ou une suspension de phosphate de calcium enchâssant l'immunogène, ou de l'hydroxyde d'aluminium adsorbant l'immunogène.

Une préparation galénique visant une réponse immunitaire mucosale administrée préférentiellement par voie nasale ou orale, mais aussi par voie vaginale ou rectale surtout pour des rappels, pourra être notamment constituée de micro sphères de polymères biodégradables, tels les PLG (poly(lactide-co-glycolides)), PLA ((poly(lactides)) et les PCL, (poly(epsilon-caprolactones)) à forme retard (Baras B. et al, Single-dose mucosal immunization with biodegradable microparticles containing a Schistosoma mansoni antigen. Infect Immun. (1999) 67:2643-8) dans lesquelles sont incluses les molécules d'antigènes, de suspensions aqueuses de phosphate de calcium enchâssant ou adsorbant l'antigène, de nanoparticules, telles les nanoparticules de chitosan.

Les préparations vaccinales pourront être conditionnées pour la voie intra nasale sous forme de gel avec comme excipient le carbopol, de gouttes nasales ou de spray et pour la voie orale sous forme de capsules gastrorésistantes, de dragées ou de granules gastrorésistants.
- Dans le cas de vaccin ADN administré par voie systémique ou mucosale, la présentation galénique du plasmide pourra être une suspension dans un liquide physiologique tel le PBS physiologique (tampon phosphate = PBS). Les plasmides pourront être inclus dans des microsphères de polymères biodégradables (PLG, PLA, PCL) et administrées dans des capsules gastrorésistantes pour ingestion (voie orale). L'ADN pourra également être exprimé dans un vecteur vivant bactérien, type salmonelle ou viral type adénovirus ou poxvirus.

La présente demande décrit aussi l'utilisation à titre d'immunogène d'un facteur qui est un facteur cytokinique ou un facteur de régulation cellulaire particulièrement transcriptionnel ou un autre type de facteur à propriétés immunosuppressives / apoptotiques / angiogéniques anormalement relâché dans le milieu extra cellulaire (stromal) par des cellules cancéreuses ou stromales de tumeurs malignes ou qui dérive d'un tel facteur.

La présente demande décrit encore l'utilisation d'un immunogène qui est un facteur ou qui dérive d'un facteur cytokinique ou d'un facteur de régulation cellulaire particulièrement transcriptionnel ou d'un autre type de facteur à propriétés immunosuppressives / apoptotiques / angiogéniques anormalement produit par des cellules cancéreuses ou stromales de tumeurs malignes pour l'obtention d'un médicament destiné à une utilisation en tant qu'anticancéreux par mécanisme de réduction des effets, sur le micro environnement desdites cellules cancéreuses ou stromales de tumeurs malignes, d'un facteur cytokinique ou d'un facteur de régulation cellulaire particulièrement transcriptionnel ou d'un autre type de facteur à propriétés immunosuppressives / apoptotiques / angiogéniques anormalement produit par lesdites cellules cancéreuses ou stromales de tumeurs malignes.

La présente demande décrit enfin un immunogène qui est un facteur ou qui dérive d'un facteur cytokinique ou d'un facteur de régulation cellulaire particulièrement transcriptionnel ou d'un autre type de facteur à propriétés immunosuppressives / apoptotiques / angiogéniques anormalement relâché dans le milieu extra cellulaire par les cellules cancéreuses ou stromales de tumeurs malignes pour son utilisation dans une méthode de traitement thérapeutique du corps humain ou animal, c'est-à-dire à titre de médicament, notamment de vaccin curatif ou préventif.

Les immunogènes décrits possèdent de très intéressantes propriétés pharmacologiques. Ils sont doués notamment de remarquables propriétés antagonistes, réductrices, inhibitrices ou notamment neutralisantes, des propriétés immunosuppressives / apoptotiques / angiogéniques de facteurs anormalement produits dans le milieu extracellulaire (stromal) par les cellules cancéreuses ou stromales de tumeurs malignes à la différence de composés de l'art antérieur qui agissent directement sur les cellules cancéreuses de tumeurs malignes.

Ces propriétés sont illustrées ci-après dans la partie expérimentale. Elles justifient l'utilisation des vaccins et immunogènes ci-dessus décrits à titre de médicament.

Les médicaments décrits trouvent leur emploi par exemple dans le traitement tant curatif que préventif de cancers d'origine épithéliale comme par exemple le cancer colorectal, le cancer de la prostate, le cancer du sein et d'origine conjonctive tels les sarcomes ou d'origine sanguine tels les lymphomes type Epstein-Barr ou les leucémies.

La présente décrit également pour objet un procédé d'immunisation active de patients caractérisé en ce que l'on utilise à titre d'immunogène un composé immunogène tel que défini ci-dessus avantageusement associé à un adjuvant d'immunité minéral, huileux ou de synthèse, ou encore un composé immunogène tel que défini ci-dessus, avantageusement couplé par exemple à l'aide d'un dialdéhyde ou associé à une protéine augmentant son immunogénicité.

Ces immunisations peuvent être réalisées tant à titre curatif qu'à titre préventif.

Les conditions préférentielles de mise en oeuvre des vaccins ci-dessus décrits s'appliquent également aux autres objets décrits ci-dessus.

Les exemples qui suivent illustrent la présente divulgation.

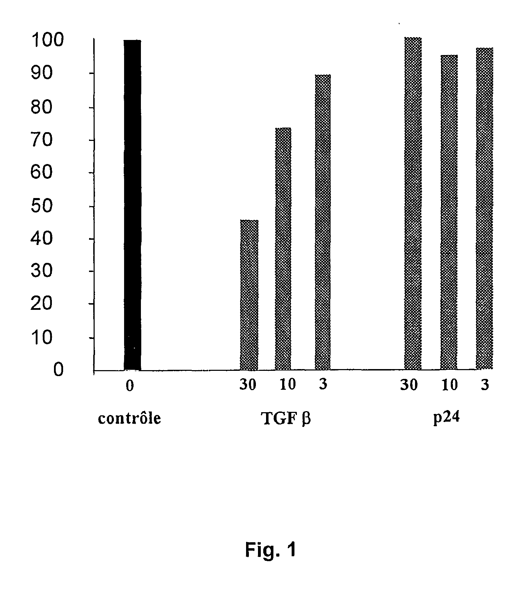

La figure 1 montre l'inhibition de la prolifération cellulaire par le TGFβ exprimée en % de prolifération cellulaire ((cpm contrôle / cpm échantillon) x 100) à trois concentrations (30, 10 et 3 ng/ml) de TGFβ et de protéine p24. Le contrôle correspond à une concentration de protéine recombinante utilisée égale à 0.

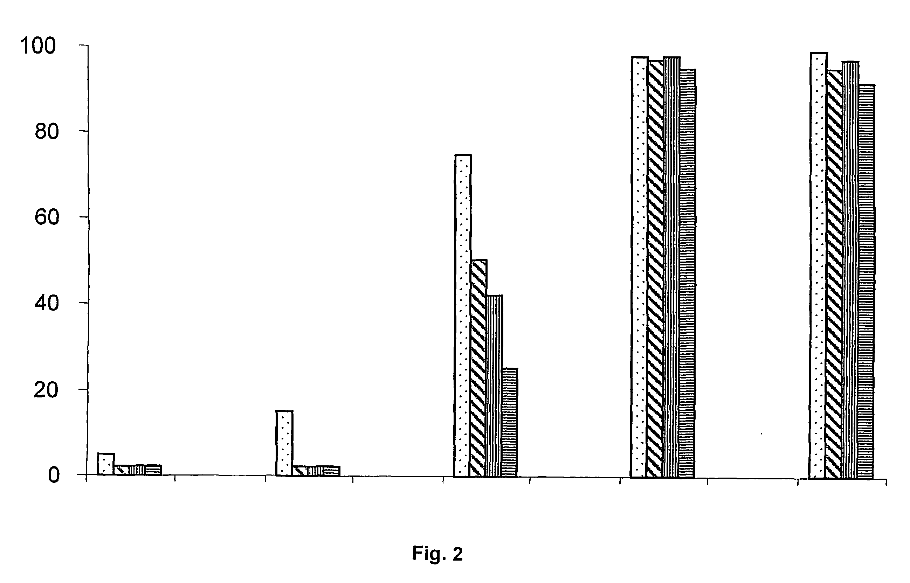

La figure 2 montre l'effet de sérum de souris immunisées avec l'immunogène VEGF non couplé ou couplé au KLH selon différentes techniques de couplage et à 4 dilutions différentes indiquées en haut du tableau. Les expériences ont été menées de gauche à droite avec des souris naïves, des souris immunisées avec du VEGF au jour J 60, des souris immunisées avec du KLH-glutaraldéhyde-VEGF pH 9 au jour J 60, des souris immunisées avec du KLH-SMCC-VEGF au jour J 60, des souris immunisées avec du KLH-SIAB-VEGF au jour J 60. Le pourcentage de neutralisation est donné en ordonnées.

### Exemple 1 : Vaccin à base de l'immunogène VEGF destinée à induire une réaction immunitaire systémique avec formation préférentielle d'anticorps spécifiques de classe IgG. (Référence)

Le vaccin est formé d'une émulsion eau dans huile constituée de 50 % d'ISA 51 (Seppic, Paris) et de 50 % d'une solution aqueuse de VEGF (20 à 200 µg/dose).

### Exemple 2 : Vaccin à base de l'immunogène plasmidique pour vaccination ADN de type systémique IL 10.

Les plasmides codant pour l'IL 10 (50 à 200 µg/dose) sont mis en suspension dans 0,2 à 1 ml de PBS pour administration intramusculaire.

### Exemple 3 : Vaccin à base de l'immunogène p53 destinée à induire une réaction immunitaire de type mucosale avec formation préférentielle d'anticorps anti p53 de classe IgA. (Référence)

L'immunogène p53 (20 à 100 µg/dose) est inclus dans un gel de phosphate de calcium en présence ou non d'adjuvant LTµ (5 à 20 µg/dose) pour l'instillation intra nasale. La préparation est administrée par voie intranasale soit sous forme de gouttes nasales, soit sous forme d'un gel par addition de carbopol.

### Exemple 4 : Vaccin à base de l'immunogène IL 10 destinée à induire une réaction immunitaire de type réaction mucosale avec formation préférentielle d'anticorps anti IL 10 de classe IgA :

On a préparé des micro sphères de PLG contenant l'immunogène (100 à 300 µg/dose) et un mutant de la toxine LT (5-25 µg/dose)

L'inclusion de l'IL 10 et du LTµ est réalisée dans les micro sphères biodégradables selon le protocole de Baras B. et al (Baras B. et al, Single-dose mucosal immunization with biodegradable microparticles containing a Schistosoma mansoni antigen. Infect Immun. (1999) 67:2643-8).

### Exemple 5: Vaccin à base de l'immunogène plasmidique IFNγ pour vaccination ADN de type mucosale. (Référence)

Les plasmides d' IFNγ (100-500 µg/dose) en présence de LTµ (5-20 µg/dose) sont inclus dans des microsphères de PLG selon le protocole décrit par Baras B. et al. L'administration par voie orale se fait par gavage ou par ingestion de capsules gastrorésistantes contenant les microsphères et un excipient à base d'alginate.

### Exemple 6 : Vaccin à base de l'immunogène VEGF destinée à induire une réaction immunitaire systémique avec formation préférentielle d'anticorps spécifiques de classe IgG. (Référence)

Le vaccin est formé d'une émulsion eau dans l'huile constituée de 50 % d'ISA 51 (SEPPIC) et de 50 % d'une solution aqueuse de VEGF (20 à 200 µg/dose).

L'immunogène provient de la préparation 3 de VEGF stabilisée par du glutaraldéhyde.

### Exemple 7 : Vaccin à base de l'immunogène VEGF couplé au KLH destinée à induire une réaction immunitaire systémique avec formation préférentielle d'anticorps spécifiques de classe IgG. (Référence)

Le vaccin est formé d'une émulsion eau dans l'huile constituée de 50 % d'ISA 51 (SEPPIC) et de 50 % d'une solution aqueuse de VEGF (20 à 200 µg/dose).

### Exemple 8 : Vaccin à base de l'immunogène E7 de HPV16 couplé au KLH pour induire une réaction immunitaire systémique. (Référence)

Le vaccin est formé d'une émulsion eau dans huile de 50 % d'ISA (SEPPIC, Paris) et de 50 % d'une solution aqueuse de E7 couplé au KLH (20 à 200 µg/dose).

Les immunogènes servant à la préparation des vaccins ci-dessus ont été préparés comme suit :

### Préparation 1 : Immunogène anti IL 6 (Référence)

Immunogène IL 6 dérivé de la cytokine recombinante IL 6 par traitement au formol suivi d'un traitement par le glutaraldéhyde :
A 1 ml d'une solution d'IL 6 à 1 mg/ml dans du tampon phosphate stérile, on ajoute 28 µl d'une solution de formol (35 %) dilué au 1/10 dans du tampon phosphate stérile. Après addition de merthiolate au 1/10.000, le mélange est placé 9 jours à l'étuve à 37°C. Il est ensuite additionné de glutaraldéhyde à la concentration 0,0026 M. Après 3 minutes, le mélange est additionné de 100 µl de glycine à 50 mg/ml pour bloquer les groupements aldéhydiques en excès et il est dialysé contre un grand volume de tampon phosphate. L'immunogène est ainsi stabilisé.

### Caractéristiques de l'IL 6

L'antigénicité de la cytokine recombinante IL 6 traitée par rapport à celle de la protéine recombinante native a été mesurée à l'aide d'un test ELISA de R&D (D6050) : la cytokine recombinante IL 6 détoxiquée présente une antigénicité égale à l'antigénicité de la protéine native correspondante.

L'absence de toxicité in vitro est mesurée par un test de prolifération cellulaire. Des cellules mononucléées du sang périphérique humain sont cultivés en présence du super antigène SEB et en présence d'une dose de la protéine recombinante IL 6 native ou détoxiquée correspondant à 10 fois et 30 fois la dose physiologique de la cytokine native. La prolifération cellulaire est exprimée en % de prolifération cellulaire [cpm (coups par minute) contrôle/cpm échantillon] x 100). Le contrôle correspond à une concentration de protéine recombinante utilisée égale à 0. Les résultats sont présentés dans le tableau suivant :

| | | % de prolifération cellulaire |
|---|---|---|
| IL 6 native | 0 ng/ml | 100 |
| IL 6 native | 30 ng/ml | 98 |
| IL 6 traitée | 30 ng/ml | 95 |

L'IL 6 traitée utilisée à des doses 10 fois et 30 fois supérieures aux doses physiologiques ne modifie pas la prolifération des cellules mononucléées du sang périphérique humain activés par le SEB.

### Préparation 2 : Immunogène anti p53 (Référence)

L'immunogène p53 a été détoxiqué par traitement au formol selon le protocole décrit par Ramon (Ramon G, Sur le pouvoir floculant et les propriétés immunisantes d'une toxine diphtérique rendue anatoxique (anatoxine). C.r. hebd. Séances Acad. Sci. (1923) 177 : 1338-1340), suivi d'un traitement au glutaraldéhyde de la protéine p53 recombinante (sc-4246, Santa Cruz) dans les conditions suivantes : à 10 µl d'une solution de p53 native à 1 mg/ml, on ajoute 3 µl d'une solution de formol dilué au 1/100 dans du tampon phosphate stérile. Le mélange est placé 2 jours à l'étuve à 37 °C. Il est ensuite additionné de 25 µl de glutaraldéhyde au 1/100. Après 15 minutes de réaction à température de laboratoire, on ajoute 2 µl de glycine 2M pour bloquer les groupements aldéhydiques en excès.

### Caractéristique de l'immunogène p53

L'antigénicité de la protéine recombinante p53 traitée par rapport à celle de la protéine recombinante native a été mesurée à l'aide d'un test ELISA d'Amersham Pharmacia Biotech (p53 Rapid Format Pantropic Human ELISA, VQIA26) : Les protéines p53 native et traitée présentent une antigénicité équivalente.

Absence de toxicité in vitro : la protéine p53 traitée utilisée à des doses dix fois et 30 fois supérieures aux doses physiologiques (0,5 à 5 ng/ml) ne modifie pas la prolifération de cellules mononucléées du sang périphérique humain activées par le SEB ou par les anticorps anti CD3. La mesure de la prolifération a été réalisée par le test à la ³H-thymidine.

### Préparation 3 : Immunogène anti VEGF (Référence)

L'immunogène VEGF dérivé de VEGF (293-VE-010 ; R&D) est obtenu par traitement à la glutaraldéhyde dans les conditions suivantes : à 100 µl d'une solution de VEGF natif à 5 µg/ml dans du tampon phosphate, on ajoute 5 µl d'une solution de glutaraldéhyde dilué au 1/500 dans du tampon phosphate stérile. Après 5 minutes de réaction à température ambiante, on ajoute 2 µl de glycine 1 M pour bloquer la réaction.

### Caractéristique du VEGF

L'antigénicité de la cytokine VEGF traitée par rapport à celle de la cytokine recombinante native a été mesurée à l'aide d'un test ELISA de R&D (DVE00) : les cytokines native et traitée présentent une antigénicité équivalente.

L'absence de toxicité in vitro a été mesurée à l'aide d'un test de prolifération cellulaire. Des cellules mononucléées du sang périphérique humain sont cultivées en présence du super antigène SEB et en présence d'une dose de la protéine recombinante VEGF native ou traitée correspondant à 10 fois et 30 fois la dose physiologique de la cytokine native. La prolifération cellulaire est exprimée en % de prolifération cellulaire ((cpm contrôle/cpm échantillon) x 100). Le contrôle correspond à une concentration de protéine recombinante utilisée égale à 0. Les résultats sont présentés dans le tableau suivant :

| | | % de prolifération cellulaire |
|---|---|---|
| VEGF natif | 0 ng/ml | 100 |
| VEGF natif | 30 ng/ml | 92 |
| VEGF traité | 30 ng/ml | 97 |

La cytokine VEGF traitée utilisée à des doses 10 fois et 30 fois supérieures aux doses physiologiques ne modifie pas la prolifération des cellules mononucléées du sang périphérique humain activées par le SEB.

### Préparation 4 : Immunogène anti TGFβ (Référence)

Immunogène TGFβ dérivé du TGFβ est détoxiqué par traitement au formol selon le protocole décrit par Ramon (Ramon G, Sur le pouvoir floculant et les propriétés immunisantes d'une toxine diphtérique rendue anatoxique (anatoxine). C.r. hebd. Séances Acad. Sci. (1923) 177 : 1338-1340), suivi d'un traitement à la glutaraldéhyde, conformément au protocole décrit pour l'immunogène p53.

### Caractéristique de l'immunogène TGFβ

L'antigénicité de la cytokine TGFβ traitée par rapport à celle de la protéine recombinante native a été mesurée à l'aide d'un test ELISA de R&D (DB100) : Les cytokines TGFβ native et traitée présentent une antigénicité équivalente.

L'absence de toxicité de la cytokine TGFβ traitée a été mesurée par un test de prolifération de cellules T décrit dans l'exemple A1. Ce test montre que le TGFβ détoxiqué utilisé à des doses physiologiques de 0,5 à 5 ng/ml ne diminue pas la prolifération des lymphocytes.

### Préparation 5 : Immunogène anti IL 10

### a) Immunogène IL 10 dérivé de l'IL 10 par traitement au formol

L'IL 10 est obtenu à partir de la protéine de fusion de l'IL 10 par traitement au formol à 37°C suivi d'un traitement court à la glutaraldéhyde, conformément au protocole décrit pour l'immunogène p53. La protéine de fusion IL 10 a été produite chez E. Coli à partir d'un ADNc cloné dans le plasmide d'expression bactérien prSetA et purifié sous forme d'une protéine de fusion avec Tag His. Cette protéine de fusion purifiée est homogène en électrophorèse d'acrylamide et en Western blot.

### Caractéristique de l'immunogène IL 10

L'antigénicité de la cytokine IL 10 traitée par rapport à celle de la protéine recombinante native a été mesurée à l'aide d'un test ELISA de R&D (D1000) : Les cytokines IL 10 native et traitée présentent une antigénicité équivalente.

L'absence de toxicité in vitro est mesurée par un test de prolifération cellulaire. La cytokine IL 10 traitée utilisée à des doses 10 fois et 30 fois supérieures aux doses physiologiques ne modifie pas la prolifération des cellules mononucléées du sang périphérique humain activées par le SEB.

### b) Immunogène plasmidique IL 10 pour vaccination d'ADN

L'immunogène plasmidique IL 10 est représenté par un ADNc de IL 10 cloné dans le plasmide d'expression bactérien prSetA.

### Préparation 6 : Vaccin anti TNFα (Référence)

### a) Immunogène TNFα dérivé du TNFα par traitement chimique

L'immunogène dérivé du TNFα (Péprotech Inc., Rocky Hill) est obtenu par traitement au formol à 37°C suivi d'un traitement court à la glutaraldéhyde, conformément au protocole décrit pour l'immunogène p53.

### Caractéristique du TNFα :

L'antigénicité de la cytokine TNFα traitée par rapport à celle de la protéine recombinante native a été mesurée à l'aide d'un test ELISA de R&D (DTA50) : Les cytokines TNFα native et traitée présentent une antigénicité équivalente.

L'absence de toxicité in vitro est mesurée par un test de prolifération cellulaire. La cytokine TNFα traitée utilisée à des doses 10 fois et 30 fois supérieures aux doses physiologiques ne modifie pas la prolifération des cellules mononucléées du sang périphérique humain activés par le SEB.

### b) Immunogène plasmidique TNFα pour vaccination ADN

L'immunogène plasmidique TNFα est représenté par un ADNc de TNFα cloné dans le plasmide d'expression bactérien prSetA.

### Préparation 7 : Immunogènes IFNγ (Référence)

### a) L'immunogène IFNγ dérivé de l' IFNγ

Cet immunogène (Péprotech Inc., Rocky Hill) est obtenu par traitement au formol à 37 °C suivi d'un traitement court à la glutaraldéhyde, conformément au protocole décrit pour l'immunogène p53.

### Caractéristique de l'immunogène :

L'antigénicité de la cytokine IFNγ traitée par rapport à celle de la protéine recombinante native a été mesurée à l'aide d'un test ELISA de R&D (DTA50) : Les cytokines IFNγ native et traitée présentent une antigénicité équivalente.

L'absence de toxicité in vitro est mesurée par un test de prolifération cellulaire. La cytokine IFNγ traitée utilisée à des doses 10 fois et 30 fois supérieures aux doses physiologiques ne modifie pas la prolifération des cellules mononucléées du sang périphérique humain activés par le SEB.

### b)Immunogène plasmidique IFNγ pour vaccination ADN

L'immunogène plasmidique IFNγ est représenté par un ADNc de l'IFNγ cloné

### Préparation 8 : Immunogène KLH-SIAB-VEGF (Référence)

Le couplage du VEGF à la protéine KLH, utilisée comme carrier a pour effet de potentialiser l'immunogénicité du VEGF.

Le couplage a été réalisé par réaction du VEGF réduit avec KLH activé par traitement par le sulfosuccinimidyl [4-iodoacétyl] aminobenzoate (appelé sulfo-SIAB).

### Etape 1 : Réduction du VEGF par le DTT

1 mg de VEGF en solution dans 500 µl de PBS a été additionné de 40 µl d'une solution de DTT (Dithiotréitol) à 50 mg/ml. Le mélange a été conservé pendant 2 heures à température ambiante, à l'abri de la lumière, et le mélange réactionnel a été filtré à travers une colonne de Sephadex G25 (1 x 15 cm) équilibrée en PBS contenant EDTA-Na₂ 5 mM, pH 7,0.

### Etape 2 : Traitement du KLH par le sulfo-SIAB

Le sulfo-SIAB est un bras écarteur qui permet de lier la protéine porteuse, ici KLH, avec l'immunogène VEGF pour faire un conjugué.

150 µl d'une solution de KLH à 20 mg/ml ont été additionnés de 50 µl de tampon borate 0,1 M - EDTA Na₂-5 mM, pH 8,5, suivi de l'addition de 20 µl d'une solution dans l'eau de sulfo-SIAB à 3,4 mg/ml, la réaction a eu lieu pendant 30 min. à température ambiante et à l'abri de la lumière, sous une barrière d'azote. Le mélange réactionnel a été alors filtré à travers une colonne de Sephadex G25 (1 x 11 cm) équilibrée avec le même tampon.

### Etape 3 : Couplage du VEGF réduit à KLH-SIAB

1 ml de solution de VEGF réduit a été mélangé avec 500 µl de KLH-SIAB. Le mélange a été incubé, à l'abri de la lumière et à température ambiante, sous azote, pendant 1 heure, puis pendant 15 heures à 4° C.

Après que la réaction a été bloquée par addition de cystéine à concentration finale 5 mM, pendant 20 min., le mélange a été purifié par chromatographie d'exclusion.

### Caractéristiques de l'immunogène KLH-SIAB-VEGF :

L'antigénicité du VEGF conjugué s'est révélée comparable à celle du VEGF isolé.

L'absence de toxicité in vitro a été mesurée par un test de prolifération cellulaire. Le conjugué KLH-SIAB-VEGF utilisé à des doses 10 fois et 30 fois supérieures aux doses physiologiques ne modifie pas la prolifération des cellules mononucléées du sang périphérique humain activés par le SEB.

### Préparation 9 : Immunogène KLH-SMCC-VEGF (Référence)

Le couplage du VEGF à la protéine KLH, utilisée comme carrier a pour effet de potentialiser l'immunogénicité du VEGF.

Le couplage a été réalisé par réaction du VEGF réduit avec le KLH activé par traitement par le (sulfosuccinimidyl [4-N-maléimidométhyl]-cyclohexane-1-carboxylate) (sulfo-SMCC).

### Etape 1 : Réduction du VEGF par le DTT

Le sulfo-SMCC est un bras écarteur qui permet de lier la protéine porteuse, ici KLH, avec le VEGF pour former un conjugué.

1 mg de VEGF en solution dans 500 µl de PBS a été additionné de 40 µl d'une solution de DTT à 50 mg/ml. Le mélange a été conservé pendant 2 heures à température ambiante, à l'abri de la lumière, et le mélange réactionnel a été filtré à travers une colonne de Sephadex G25 (1 x 15 cm) équilibrée en PBS contenant EDTA-Na₂ 5 mM, pH 7,0.

### Etape 2 : Traitement du KLH par le sulfo-SMCC

150 µl d'une solution de KLH à 20 mg/ml ont été additionnés de 50 µl de tampon borate 0,1 M - EDTA Na₂-5 mM, pH 8,5, suivi de l'addition de 20 µl d'une solution dans l'eau de sulfo-SMCC à 3,4 mg/ml, la réaction a eu lieu pendant 30 min. à température ambiante et à l'abri de la lumière, sous une barrière d'azote. Le mélange réactionnel a été alors filtré à travers une colonne de Sephadex G25 (1 x 11 cm) équilibrée avec le même tampon.

### Etape 3 : Couplage de VEGF réduit à KLH-SMCC

1 ml de solution de VEGF réduit a été mélangé avec 500 µl de KLH-SMCC. Le mélange a été incubé, à l'abri de la lumière et à température ambiante, sous azote, pendant 1 heure, puis pendant 15 heures à 4° C.

Après que la réaction a été bloquée par addition de cystéine à concentration finale 5 mM, pendant 20 min., le mélange a été purifié par chromatographie d'exclusion.

### Caractéristique de l'immunogène KLH-SMCC-VEGF :

L'antigénicité de VEGF conjugué s'est révélée comparable à celle du VEGF isolé.

L'absence de toxicité in vitro est mesurée par un test de prolifération cellulaire. le conjugué KLH-SMCC-VEGF utilisé à des doses 10 fois et 30 fois supérieures aux doses physiologiques ne modifie pas la prolifération des cellules mononucléées du sang périphérique humain activés par le SEB.

### Préparation 10 : Immunogène KLH-glutaraldéhyde-VEGF (Référence)

Le couplage a pour effet de potentialiser l'immunogénicité de la protéine VEGF.

Le couplage a été réalisé par réaction de la molécule VEGF avec KLH activé par la glutaraldéhyde.

1 ml de solution de KLH à 10 mg/ml dans PBS a été activé par dialyse contre 100 ml d'une solution de glutaraldéhyde à 0,2 % dans PBS, pendant une nuit, à 4° C. L'excès de glutaraldéhyde a été éliminé par dialyse de la protéine activée contre 3 changements de 200 ml de PBS de 2 heures chacun.

A 400 µl de KLH activé (4 mg) sont ajoutés 1 mg d'une solution à 1 mg/ml de la protéine VEGF dans PBS et le mélange réactionnel est agité, pendant 1 nuit, à 4° C. Les groupements aldéhydiques libres sont alors bloqués par réaction pendant 1 heure avec 100 µl de glycine 2.5 M et le mélange est purifié par chromatographie d'exclusion. L'antigénicité de la protéine VEGF dans le conjugué s'est révélée légèrement supérieure à celle de VEGF isolé.

### Préparation 11 : Immunogène KLH- glutaraldéhyde -E7 (Référence)

Le couplage a pour effet de potentialiser l'immunogénicité de la protéine E7.

Le couplage a été réalisé par réaction de la molécule E7 avec KLH activé par la glutaraldéhyde à partir d'1 ml de solution de KLH à 10 mg/ml dans du PBS selon le même protocole que celui décrit pour la préparation 10.

L'antigénicité de la protéine E7 dans le conjugué s'est révélée légèrement supérieure à celle de E7 isolé.

### Préparation 12 : KLH- glutaraldéhyde -IFNα. (Référence)

L'IFNα a été conjugué au KLH dans les mêmes conditions que celles décrites dans la préparation 11 pour la protéine E7.

L'antigénicité de l'IFNα conjugué s'est révélée légèrement supérieure à celle de l'IFNα traité au glutaraldéhyde seul.

### Etude pharmacologique (Référence)

A - Présence dans le milieu extracellulaire de tumeurs malignes, de molécules participant à l'immunosuppression, l'apoptose ou l'angiogénèse du micro environnement des cellules cancéreuses.

### Expérimentation A1 :

La protéine p53 qui s'accumule dans les tumeurs malignes et est présente dans les milieux extracellulaires dont le sérum (Zusman I, Sandler B, Gurevich P, Zusman R, Smirnoff P, Tendler Y, Bass D, Shani A, Idelevich E, Pfefferman R, Davidovich B, Huszar M, Glick J. Comparative study of the role of sérum levels of p53 antigen and its tumor cell concentration in colon cancer detection. Hum Antibodies Hybridomas. (1996) :123-8), active la surproduction par les APC d'IFNα, médiateur de l'immunosuppression, et de TNFα, cytokine participant à l'expression des molécules d'adhérence des cellules endothéliales et à l'apoptose des cellules immunitaires.

### Protocole expérimental

Des macrophages, qui proviennent de la différentiation de monocytes élutriés cultivés pendant 5 jours dans des poches de téflon en présence de GMC-SF (F. Sallusto et al, Efficient presentation of soluble antigen by cultured human dendritic cells is maintained by granulocyte/macrophage colony-stimulating factor plus interleukin 4 and downregulated by tumor necrosis factor alpha. J Exp Med (1994) 179, 1109), sont activés avec du LPS pendant 16 heures. Ces macrophages ainsi activés sont ensuite cultivés en présence de doses croissantes (0 - 10 µg/ml) de la protéine recombinante p53 native (sc-4246, Santa Cruz) et de protéine contrôle) dans du milieu sans sérum pendant 24 heures. La protéine contrôle a été la protéine recombinante p24 (protéine du HIV-1, origine ANRS)

### Résultats :

La mesure de la surproduction APC d'IFNα et de TNFα dans les surnageants (SN) de culture des APCs s'effectue respectivement par le test biologique standard de l'IFNα, utilisant la lyse des cellules MDBK par le VSV (S. Rubinstein et al, Convenient assay for interferons. J. Virol (1981) 37, 755) et par un test ELISA de R&D (DTA50, R&D).

Le titre d'IFNα dans les surnageant correspond à l'inverse de la plus forte dilution des surnageants induisant 50 % de protection des cellules contre l'effet cytopathique du VSV. La mesure de TNFα dans les surnageants est réalisée en suivant le protocole décrit par le producteur et est exprimée en µg/ml. Les résultats sont représentés dans le tableau suivant

| p53 expérimental | Titre d'IFNα (dilution⁻¹) | TNFα (pg/ml) |
|---|---|---|
| 10 µg/ml | 128 | 8700 |
| 3 µg/ml | 64 | 6200 |
| 1 µg/ml | 32 | 5600 |
| 0,3 µg/ml | 12 | 2600 |
| 0,1 µg/ml | 6 | 1300 |
| 00 µg/ml | 2 | 200 |

| p24 contrôle | Titre d'IFNα (dilution⁻¹) | TNFα (pg/ml) |
|---|---|---|
| 10 µg/ml | 2 | 250 |
| 3 µg/ml | 2 | 200 |
| 1 µg/ml | 2 | 200 |
| 0,3 µg/ml | 2 | 200 |
| 0,1 µg/ml | 2 | 200 |
| 00 µg/ml | 2 | 200 |

La protéine recombinante p53 native induit la surproduction d'IFNα et de TNFα, alors que la protéine recombinante p24 utilisée dans les contrôles n'induit aucune synthèse.

Un lysat de culture de cellules d'insectes à baculovirus exprimant la protéine p53 a donné des résultats similaires à ceux décrits pour la protéine recombinante p53 produite chez E. Coli.

### Expérimentation A2 :

La cytokine TGFβ, relâchée dans le milieu extracellulaire par des cellules cancéreuses inhibe la prolifération de cellules T et active la production par les macrophages de l'IFNα, cytokine immunosuppressive majeure.

### Protocole expérimental

Des cellules mononucléées du sang périphérique humain, isolées sur gradient de Ficoll à partir du sang périphérique de sujet sain, sont cultivés en présence de l'anticorps anti-CD3 et en présence de doses croissantes (0-30 ng/ml) de la protéine recombinante TGFβ active (240-B-002, R&D) et de doses croissantes (0-30 µg/ml) d'une protéine contrôle, la protéine recombinante p24.

L'inhibition de la prolifération des cellules T est mesurée à l'aide d'un test de prolifération cellulaire (Lachgar A., Bernard J., Bizzini B., Astgen A., Le Coq H., Fouchard M., Chams V., Feldman M., Richardson M., Rappaport J., Burny A. & J.F. Zagury : Repair of the in vitro HIV-1-induced immunosuppression and blockade of the generation of functional suppressive CD8 cells by anti-alpha interferon and anti-Tat antibodies. Biomed & Pharmacother. (1996) 50:13-18).

L'activation de la production d'IFNα par les macrophages est mesurée selon le protocole décrit dans l'expérimentation A1. Les macrophages activés sont cultivés en présence de doses croissantes (0-1 µg/ml) de la protéine recombinante TGFβ active et d'une protéine contrôle, la protéine recombinante p24, dans du milieu sans sérum pendant 24 heures.

### Résultats :

Inhibition de la prolifération cellulaire par le TGFβ :
La prolifération cellulaire est exprimée en % de prolifération cellulaire ((cpm contrôle / cpm échantillon) x 100) à trois concentrations (30, 10 et 3 µg/ml) de TGFβ et de protéine p24. Le contrôle correspond à une concentration de protéine recombinante utilisée égale à 0. Les résultats sont présentés dans la figure 1 :
   Ces résultats montrent que la prolifération cellulaire est diminuée de manière dose dépendante par le TGFβ actif, alors qu'elle ne l'est pas par la p24.

Activation par le TGFβ de la surproduction d'IFNα par les macrophages.

Le titre d'IFNα dans les surnageants correspond à l'inverse de la plus forte dilution des surnageants induisant 50 % de protection contre l'effet cytopathique du VSV. Les résultats sont présentés dans le tableau suivant :

| TGFβ expérimental | Titre d'IFNα |
|---|---|
| 1 µg/ml | 16 |
| 300 ng/ml | 8 |
| 100 ng/ml | 4 |
| 30 ng/ml | 2 |
| 00 ng/ml | 0 |

| P24 contrôle | Titre d'IFNα |
|---|---|
| 1 µg/ml | 0 |
| 300 ng/ml | 0 |
| 100 ng/ml | 0 |
| 30 ng/ml | 0 |
| 00 µg/ml | 0 |

La protéine recombinante TGFβ active induit la surproduction d'IFNα, alors que la protéine recombinante p24 n'induit aucune synthèse.

### Expérience de vaccination 1 :

Vaccination anti-IL 10 de la souris pour l'induction d'une immunité systémique et mucosale avec formation préférentielle d'anticorps spécifiques de classes IgG et IgA.

### Protocole d'immunisation

- Jour 0 : Injection IM d'une suspension d'immunogène plasmidique exprimant l'IL 10 (100 µg) dans 0,2 ml de PBS préparée à l'exemple 2.
- Jour 7, jour 8, jour 9 : Administration par gavage de suspensions aqueuses de micro sphères (PLGA) incluant l'immunogène IL 10 (100 µg/dose) et l'adjuvant LTµ (5 µg/dose).

Les souris contrôles reçoivent les mêmes préparations sans immunogène.

### Suivi :

On sacrifie les animaux 15 jours après la dernière immunisation et on constate l'absence de toxicité (mesurée par l'absence de signes cliniques (comportement ; poils ; poids) et par examen anatomique après autopsie.

Réaction immunitaire testée par la présence dans le sérum d'anticorps de type IgG et IgA , mesurée par ELISA et exprimée par la densité optique 15 jours après le dernier gavage.

| | Souris contrôle (D.O) | Souris immunisées (D.O) |
|---|---|---|
| Ac anti IL 10 classe IgG | 0,2 | 0,920 |
| Ac anti IL 10 classe IgA | 0,1 | 0,780 |

### Résultats :

Innocuité clinique et absence de lésions anatomiques.

Présence d'anticorps (Ac) anti IL 10 de type IgG et de type IgA dans le sérum

### Expérience de vaccination 2 : (Référence)

Vaccination anti-VEGF de la souris par l'induction d'une immunité systémique et mucosale avec formation préférentielle d'anticorps spécifique de classe IgG et IgA.

### Protocole d'immunisation

- Jour 0 : Injection IM d'une suspension d'immunogène VEGF (20 µg) dans l'ISA 51 préparée à l'exemple 1.
- Jour 7, jour 14, jour 21 : Administration intranasale à l'aide de pipette Hamilton de 10 µl d'une suspension aqueuse contenant 20 µg d'immunogène et 5 µg de LTµ enchâssés dans un gel de phosphate de calcium.

Les souris contrôles reçoivent les mêmes préparations sans immunogène

### Suivi :

On sacrifie les animaux 15 jours après la dernière immunisation et on constate l'absence de toxicité (mesurée par l'absence de signes cliniques (comportement ; poils ; poids) et par examen anatomique après autopsie.

Réaction immunitaire testée par la présence dans le sérum d'anticorps de type IgG et IgA , mesurée par ELISA et exprimée par la densité optique 15 jours après la dernière instillation.

| | Souris contrôle (D.O) | Souris immunisées (D.O) |
|---|---|---|
| Ac anti VEGF classe IgG | 0,27 | 1,64 |
| Ac anti VEGF classe IgA | 0,15 | 1,118 |

### Résultats :

Innocuité clinique et absence de lésions anatomiques.

Présence d'anticorps anti VEGF de type IgG et de type IgA dans le sérum.

### Expérience de vaccination 3 : (Référence)

Vaccination anti p53 de la souris par l'induction d'une immunité systémique et mucosale avec formation préférentielle d'anticorps spécifique de classe IgG et IgA.

### Protocole d'immunisation

- Jour 0 : Injection IM d'une suspension d'immunogène p53 (20 µg) dans l'ISA 51 préparée comme à l'exemple 1.
- Jour 7, jour 14, jour 21 : Administration intra nasale à l'aide de pipette Hamilton de 10 µl d'une suspension aqueuse contenant 20 µg d'immunogène et 5 µg de LTµ enchâssés dans un gel de phosphate de calcium.

Les souris contrôles reçoivent les mêmes préparations sans immunogène

### Suivi :

On sacrifie les animaux 15 jours après la dernière immunisation et on constate l'absence de toxicité (mesurée par l'absence de signes cliniques (comportement ; poils ; poids) et par examen anatomique après autopsie.

Réaction immunitaire testée par la présence dans le sérum et dans la salive d'anticorps de type IgG et IgA , mesurée par ELISA et exprimée par la densité optique 15 jours après la dernière instillation.

### Résultats :

Innocuité clinique et absence de lésions anatomiques.

Présence d'anticorps anti p53 de type IgG et de type IgA dans le sérum et dans la salive.

| sérum | Souris contrôle (D.O) | Souris immunisées (D.O) |
|---|---|---|
| Ac anti p53 classe IgG | 0,184 | 1,492 |
| Ac anti p53 classe IgA | 0,208 | 1,071 |

| salive | Souris contrôle (D.O) | Souris immunisées (D.O) |
|---|---|---|
| Ac anti p53 classe IgG | 0,184 | 1,5 |
| Ac anti p53 classe IgA | 0,208 | 0,980 |

### Expérience de vaccination 4 : (Référence)

Vaccination anti IL 6 de la souris pour l'induction d'une immunité systémique avec formation d'anticorps spécifiques de classe IgG

### Protocole d'immunisation

- Jour 0 : Injection IM d'une suspension d'immunogène IL 6 (20 µg) dans l'ISA 51 préparée comme à l'exemple 1.
- Jour 21 : Rappel par voie IM d'une émulsion d'IL 6 (5 µg) dans l'ISA 51

Les souris contrôles reçoivent les mêmes préparations sans immunogène

### Suivi :

On sacrifie les animaux 15 jours après le rappel et on constate l'absence de toxicité (mesurée par l'absence de signes cliniques (comportement ; poils ; poids) et par examen anatomique après autopsie.

Réaction immunitaire testée par la présence dans le sérum d'anticorps de type IgG et IgA , mesurée par ELISA et exprimée par la densité optique 7 jours après le rappel.

| | Souris contrôle (D.O) | Souris immunisées (D.O) |
|---|---|---|
| Ac anti IL 6 classe IgG | 0,280 | 2,356 |
| Ac anti IL 6 classe IgA | 0,230 | 0,320 |

### Résultats :

Innocuité clinique et absence de lésions anatomiques.

Présence d'anticorps anti IL 6 de type IgG dans le sérum.

### Expérience de vaccination 5 : (Référence)

Vaccination anti IL 6 de la souris par l'induction d'une immunité systémique et mucosale avec formation préférentielle d'anticorps spécifique de classe IgG et IgA.

### Protocole d'immunisation

- Jour 0 : Injection IM d'une suspension d'immunogène IL 6 (20 µg) dans l'ISA 51 préparée comme à l'exemple 1.
- Jour 7, jour 8, jour 9 : Administration par gavage de micro sphères de PLG contenant l'immunogène (100 µg/dose) et l'adjuvant LTµ (5 µg/dose).

Les souris contrôles reçoivent les mêmes préparations sans immunogène

### Suivi :

On sacrifie les animaux 15 jours après le dernier gavage et on constate l'absence de toxicité (mesurée par l'absence de signes cliniques (comportement ; poils ; poids) et par examen anatomique après autopsie.

Réaction immunitaire testée par la présence dans le sérum d'anticorps de type IgG et IgA , mesurée par ELISA et exprimée par la densité optique 15 jours après le dernier gavage.

| | Souris contrôle (D.O) | Souris immunisées (D.O) |
|---|---|---|
| Ac anti IL 6 classe IgG | 0,250 | 1,400 |
| Ac anti IL 6 classe IgA | 0,175 | 1,62 |

### Résultats :

Innocuité clinique et absence de lésions anatomiques.

Présence d'anticorps anti IL 6 de type IgG et de type IgA dans le sérum.

### Exemple de vaccination 6 : vaccination anti-VEGF par le conjugué KLH-SIAB-VEGF. (Référence)

L'activité immunogénique (humorale) du conjugué KLH-SIAB-VEGF par rapport à celle du VEGF natif a été étudiée chez la souris balb c de 18-20 g.

Au jour J 0, un groupe de 3 souris reçoit une injection de 0,2 ml (50 µg) d'une émulsion en adjuvant complet de Freund par voie intramusculaire. Une injection de rappel de 5 µg en adjuvant incomplet de Freund est donnée à J 21 et J 60.

Un prélèvement sanguin au niveau rétro-orbital est effectué sur chaque souris avant la première injection à J -2
3 souris contrôles reçoivent les mêmes préparations sans immunogène.
3 souris reçoivent 100 µg de la préparation et l'absence de symptômes de maladie est étudiée pendant les 7 jours suivant l'injection.

Les souris sont sacrifiées 12 jours après la dernière immunisation.

L'absence de toxicité est mesurée par l'absence de signes cliniques : (comportement, poils, poids) et par examen anatomique après autopsie.

### Résultats :

Aucune des 3 souris immunisées avec 100 µg de la préparation ne manifeste de symptômes de maladie pendant les 7 jours suivant l'injection.

Les souris immunisées aussi bien par le conjugué KLH-SIAB-VEGF que par le VEGF uniquement ne présentent aucun signe clinique et aucune lésions anatomiques.

La réaction immunitaire est mesurée par :
a) la présence dans le sérum d'anticorps de type IgG dirigés contre la protéine recombinante VEGF native, mesurée par ELISA et exprimée en titre (inverse de la dilution donnant une densité optique supérieure à 0.3)

| | Titre | |
|---|---|---|
| | J -2 | J 72 |
| souris contrôle : | | |
| souris 1 | <500⁻¹ | <500⁻¹ |
| souris 2 | <500⁻¹ | <500⁻¹ |
| souris 3 | <500⁻¹ | <500⁻¹ |

| Souris immunisées avec le VEGF : | | |
|---|---|---|
| souris 4 | <500⁻¹ | 500⁻¹ |
| souris 5 | <500⁻¹ | 1000⁻¹ |
| souris 6 | <500⁻¹ | 750⁻¹ |

| Souris immunisées avec le conjugué KLH-SIAB-VEGF : | | |
|---|---|---|
| souris 7 | <500⁻¹ | >64000⁻¹ |
| souris 8 | <500⁻¹ | >64000⁻¹ |
| souris 9 | <500⁻¹ | >64000⁻¹ |

Les souris immunisées avec le conjugué KLH-SIAB-VEGF présentent des titres d'anticorps de type IgG anti-VEGF plus importants que ceux des souris immunisées avec le VEGF uniquement.

### Exemple 7 : comparaison des activités neutralisantes des sérums de souris immunisées par le conjugué KLH-SIAB-VEGF ou par du VEGF natif.

### (Référence)

L'activité neutralisante de ces anticorps a été mesurée à l'aide du test biologique standard de l'activité du VEGF. Différentes dilutions de sérums (1/100 - 1/800) prélevés à J -2 et J 72 sont incubées pendant 2 heures avec 10 ng/ml de VEGF natif. Ces dilutions sont ensuite déposées sur des cellules endothéliales (HUVEC) cultivées dans des puits à fond plat d'une plaque de micro-culture à raison de 3000 cellules/puits. La culture cellulaire est poursuivie à 37 °C en atmosphère humide chargée à 5 % de CO₂ pendant 6 jours. 18 heures avant la fin de l'incubation, 0.5 µCi de thymidine tritiée / puits sont ajoutés.

Les résultats sont donnés en % de neutralisation.

| | | % de neutralisation | | | |
|---|---|---|---|---|---|
| | | 1/100 | 1/200 | 1/400 | 1/800 |
| Souris immunisées avec le VEGF: | | | | | |
| souris 4 | J -2 | 0 | 0 | 0 | 0 |
| | J 72 | 15 | 0 | 0 | 0 |
| souris 5 | J -2 | 0 | 0 | 0 | 0 |
| | J 72 | 20 | 0 | 0 | 0 |
| souris 6 | J -2 | 0 | 0 | 0 | 0 |
| | J 72 | 15 | 0 | 0 | 0 |

| Souris immunisées avec le KLH-VEGF | | | | | |
|---|---|---|---|---|---|
| souris 7 | J -2 | 0 | 0 | 0 | 0 |
| | J 72 | 100 | 100 | 100 | 100 |
| souris 8 | J -2 | 0 | 0 | 0 | 0 |
| | J 72 | 100 | 100 | 100 | 100 |
| souris 9 | J -2 | 0 | 0 | 0 | 0 |
| | J 72 | 100 | 100 | 100 | 100 |

Les anticorps induits par le conjugué KLH-VEGF ont un pouvoir neutralisant plus important que celui des anticorps induits par le VEGF.

### Exemple 9 : Comparaison des activités neutralisantes des souris immunisées avec soit le VEGF natif, soit le conjugué KLH-SIAB-VEGF, soit le conjugué KLH-SMCC-VEGF, soit le KLH-gluta-VEGF. (Référence)

L'activité neutralisante est déterminée selon le même protocole expérimental que celui décrit dans l'exemple 8. Comme dans le protocole d'expérimentation de l'exemple 7, les souris ont été immunisées à J 0, J 21 et à J 60.

La figure 2 résume les résultats obtenus : On voit que dès J 30, des neutralisations importantes apparaissent pour les conjugués KLH-SIAB-VEGF et KLH-SMCC-VEGF. Il faut- attendre J 70 pour obtenir des neutralisations avec le conjugué KLH-glutaraldéhyde-VEGF. Par contre, on n'observe pas de neutralisation pour le VEGF natif.

### Exemple 10 : vaccination avec le conjugué KLH - glutaraldéhyde - E7.

### (Référence)

L'activité immunogénique (humorale et cellulaire) du conjugué KLH-E7 par rapport à celle de la protéine E7 a été étudiée chez la souris balb c de 18-20 g.

Au jour J 0, un groupe de 3 souris reçoit une injection de 0,2 ml (50 µg) d'une émulsion en ACF par voie intramusculaire. Une injection de rappel de 5 µg en AIF est donnée à J 21 et J 60.

Un prélèvement sanguin au niveau rétro-orbital est effectué sur chaque souris avant la première injection à J -2
3 souris contrôles reçoivent les mêmes préparations sans immunogène.
3 souris reçoivent 100 µg de la préparation et l'absence de symptômes de maladie est étudiée pendant les 7 jours suivant l'injection.

Les souris sont sacrifiées 12 jours après la dernière immunisation.

L'absence de toxicité est mesurée par l'absence de signes cliniques : (comportement, poils, poids) et par examen anatomique après autopsie.

### Résultats :

Aucune des 3 souris immunisées avec 100 µg de la préparation ne manifeste de symptômes de maladie pendant les 7 jours suivant l'injection.

Les souris immunisées aussi bien par le conjugué KLH-E7 que par la protéine E7 uniquement ne présentent aucun signe clinique et aucune lésion anatomique.

La réaction immunitaire est mesurée par :
1- la présence dans le sérum d'anticorps de type IgG dirigée contre la protéine recombinante E7 native, mesurée par ELISA et exprimée en titre (inverse de la dilution donnant une densité optique supérieure à 0.3)

| | Titre | |
|---|---|---|
| | J -2 | J 72 |
| souris contrôle : | | |
| souris 1 | <500⁻¹ | <500⁻¹ |
| souris 2 | <500⁻¹ | <500⁻¹ |
| souris 3 | <500⁻¹ | <500⁻¹ |

| souris immunisées avec la protéine E7 : | | |
|---|---|---|
| souris 4 | <500⁻¹ | 32 000⁻¹ |
| souris 5 | <500⁻¹ | 64 000⁻¹ |
| souris 6 | <500⁻¹ | 48 000⁻¹ |

| souris immunisées avec le conjugué KLH-E7 : | | |
|---|---|---|
| souris 7 | <500⁻¹ | >64 000⁻¹ |
| souris 8 | <500⁻¹ | >64 000⁻¹ |
| souris 9 | <500⁻¹ | >64 000⁻¹ |

Les souris immunisées avec le conjugué KLH-E7 présentent des titres d'anticorps de type IgG anti-E7 plus importants que ceux des souris immunisées avec la protéine E7 uniquement.

## Revendications

1. Un vaccin **caractérisé en ce qu'**il renferme à titre de principe actif un immunogène qui est l'IL-10 ou qui dérive de l'IL-10 par traitement chimique, physique, par mutation génétique, par conditionnement adjuvant, ou est un plasmide comprenant un gène promoteur d'expression et le gène codant l'IL-10 ou un immunogène dérivant de l'IL-10 par mutation génétique, ou est un fragment protéique ou peptidique de 8 à 110 acides aminés d'un tel facteur IL10 ainsi qu'un excipient pharmaceutiquement acceptable permettant l'induction d'une réaction immunitaire systémique ou mucosale avec formation d'anticorps neutralisants de classe IgG ou IgA sécrétoire dirigés contre le facteur IL10 natif.

2. Un vaccin selon la revendication 1 **caractérisé en ce que** l'immunogène dérive de l'IL10 par couplage à une protéine porteuse qui est le KLH.

3. Un vaccin selon l'une des revendications 1 et 2 **caractérisé en ce qu'**il renferme à titre de principe actif un immunogène qui est un mutant du facteur natif IL10 ou un fragment du facteur natif IL10.

4. Utilisation d'un immunogène qui est l'IL10 ou qui dérive de l'IL10 par traitement chimique, physique, par mutation génétique, par conditionnement adjuvant, ou est un plasmide comprenant un gène promoteur d'expression et le gène codant l'IL-10 ou un immunogène dérivant de l'IL-10 par mutation génétique, ou est un fragment protéique ou peptidique de 8 à 110 acides aminés d'un tel facteur IL10 pour l'obtention d'un médicament destiné à une utilisation en tant qu'anticancéreux par mécanisme de réduction des effets, sur le micro environnement desdites cellules cancéreuses ou stromales de tumeurs malignes, du facteur cytokinique IL10.

## Patentansprüche

1. Impfstoff, **dadurch gekennzeichnet, dass** er als Wirkstoff ein Immunogen, bei dem es sich um IL-10 handelt oder das durch chemische oder physikalische Behandlung, durch genetische Mutation, durch Adjuvanskonditionierung von IL-10 abgeleitet ist oder bei dem es sich um ein Plasmid handelt, das ein Expressionspromotor-Gen und das Gen, das IL-10 oder ein Immunogen, das durch genetische Mutation von IL-10 abgeleitet ist, codiert, umfasst, oder es sich um ein Protein- oder Peptidfragment von 8 bis 110 Aminosäuren eines solchen Faktors IL-10 handelt, sowie einen pharmazeutisch annehmbaren Arzneimittelhilfsstoff, der die Induktion einer systemischen oder mukosalen Immunreaktion mit Bildung von neutralisierenden Antikörpern der Klasse sezerniertes IgG oder IgA, die gegen den nativen Faktor IL-10 gerichtet sind, ermöglicht, umfasst.

2. Impfstoff gemäß Anspruch 1, **dadurch gekennzeichnet, dass** das Immunogen durch Kopplung an ein Trägerprotein, bei dem es sich um KLH handelt, von IL-10 abgeleitet ist.

3. Impfstoff gemäß einem der Ansprüche 1 und 2, **dadurch gekennzeichnet, dass** er als Wirkstoff ein Immunogen, bei dem es sich um eine Mutante des nativen Faktors IL-10 oder ein Fragment des nativen Faktors IL-10 handelt, umfasst.

4. Verwendung eines Immunogens, bei dem es sich um IL-10 handelt oder das durch chemische oder physikalische Behandlung, durch genetische Mutation, durch Adjuvanskonditionierung von IL-10 abgeleitet ist oder bei dem es sich um ein Plasmid handelt, das ein Expressionspromotor-Gen und das Gen, das IL-10 oder ein Immunogen, das durch genetische Mutation von IL-10 abgeleitet ist, codiert, umfasst, oder es sich um ein Protein- oder Peptidfragment von 8 bis 110 Aminosäuren eines solchen Faktors IL-10 handelt, zur Gewinnung eines Medikaments zur Verwendung als Antikrebsmedikament über einen Mechanismus der Reduktion der Wirkungen des Cytokinfaktors IL-10 auf die Mikroumgebung der Krebs- oder Stromazellen von malignen Tumoren.

## Claims

1. A vaccine **characterized in that** it contains as active ingredient an immunogen which is IL-10 or is derived from IL-10 by chemical, physical treatment by genetic mutation, by adjuvant conditioning, or is a plasmid comprising an expression promoter gene and the gene coding IL-10 or an immunogen derived from IL-10 by genetic mutation, or is a proteinic or peptidic fragment of 8 to 110 amino acids of such IL-10 factor as well as a pharmaceutically acceptable excipient allowing the induction of an systemic or mucosal immune reaction with formation of neutralizing antibodies of IgG or secretory IgA class directed against the native IL-10 factor.

2. A vaccine according to claim 1 **characterized in that** the immunogen derived from IL-10 by conjugation to a carrier protein which is KLH.

3. A vaccine as claimed in anyone of claims 1 and 2 **characterized in that** it contains as active ingredient an immunogen which is a mutant of the native IL-10 factor or a fragment of the native IL-10 factor.

4. Use of an immunogen which is IL-10 or is derived from IL-10 by chemical, physical treatment by genetic mutation, by adjuvant conditioning, or is a plasmid comprising an expression promoter gene and the gene coding IL-10 or an immunogen derived from IL-10 by genetic mutation, or is a proteinic or peptidic fragment of 8 to 110 amino acids of such IL-10 factor for obtaining a medicament intended for a use as an anticancer medicament by a mechanism of reducing the effects, on the microenvironment of said cancerous cells or stromal cells of malignant tumors, of IL-10 cytokinic factor.
